# EUROPEAN PATENT APPLICATION

(11) **EP 1 905 434 A1**
(43) Date of publication of application: **02.04.2008**
(21) Application number: 06121276.7
(22) Date of filing: 26.09.2006
(51) Int. Cl.: A61K 31/135, A61K 47/38, A61P 37/06

(54) **Organic compounds comprising an S1P receptor agonist and their therapeutic use**

(71) Applicant: Novartis AG, 4056 Basel (CH)
(72) Inventor: The designation of the inventor has not yet been filed
(74) Representative: Vögeli-Lange, Regina

(57) **Abstract**

The present invention provides a solid pharmaceutical composition suitable for oral administration, comprising an S1P receptor agonist in the absence of a sugar alcohol.

## Description

The present invention relates to pharmaceutical compositions comprising a sphingosine-1 phosphate receptor agonist and/or modulator.

Sphingosine-1 phosphate (hereinafter "S1P") is a natural serum lipid. Presently there are 8 known S1P receptors, namely S1P1 to S1P8. S1P receptor agonists have accelerating lymphocyte homing properties.

S1P receptor agonists are immunomodulating compounds which elicit a lymphopenia resulting from a re-distribution, preferably reversible, of lymphocytes from circulation to secondary lymphatic tissue, evoking a generalized immunosuppression. Naive cells are sequestered, CD4 and CD8 T-cells and B-cells from the blood are stimulated to migrate into lymph nodes (LN) and Peyer's patches (PP), and thus infiltration of cells into transplanted organs is inhibited.

The various known S1P receptor agonists show structural similarities, which result in related problems in providing a suitable formulation. In particular, there is a need for an S1P receptor agonist containing formulation which is well-adapted for oral administration in a solid form, e.g. as a tablet or capsule.

Accordingly, the present invention provides a solid pharmaceutical composition suitable for oral administration, comprising a S1P receptor agonist and a microcrystalline cellulose, in the absence of a sugar alcohol.

It has surprisingly been found that solid compositions comprising a microcrystalline cellulose provide formulations which are particularly well suited to the oral administration of S1P receptor agonists.

The compositions provide a convenient means of systemic administration of S1P receptor agonists, do not suffer from the disadvantages of liquid formulations for injection or oral use, and have good physicochemical and storage properties. In particular, the compositions of the present invention may show a high level of uniformity in the distribution of the S1P receptor agonist throughout the composition, as well as high stability. The compositions of the invention may be manufactured on high speed automated equipment, and thus do not require hand encapsulation.
The composition of the present invention, comprising a S1P receptor agonist and a microcrystalline cellulose, in the absence of a sugar alcohol. may further comprise a lubricant.

Suitable lubricants include stearic acid, magnesium stearate, calcium stearate, zinc stearate, glyceryl palmitostearate, sodium stearyl fumarate, canola oil, hydrogenated vegetable oil such as hydrogenated castor oil (e.g. Cutina® or Lubriwax® 101), mineral oil, sodium lauryl sulfate, magnesium oxide, colloidal silicon dioxide, polyethylene glycol, polyvinyl alcohol, sodium benzoate, talc, poloxamer, or a mixture of any of the above.

Preferably the lubricant comprises magnesium stearate or a hydrogenated vegetable oil.

The composition preferably contains 0.01 to 5% by weight of the lubricant, more preferably 1 to 3% by weight, e.g. about 2% by weight, based on the total weight of the composition.

The composition may comprise one or more further excipients such as carriers, binders or diluents.

The composition may comprise an additional binder for example, methylcellulose, hydroxypropylcellulose, hydroxypropylmethylcellulose, dicalcium phosphate, PVP e.g. cellulose, polyethylene glycols, polyvinylpyrrolidone, starch mucilage, acacia, alginic acid, carboxymethylcellulose, hydroxyethylcellulose" dextrin, ethylcellulose, gelatin, guar gum, hydroxypropylmethylcellulose, magnesium aluminium silicate, kaltodectrin, methylcellulose, polyethylene oxide, povidone, sodium alginate and hydrogenated vegetable oils.

The composition may also include, or alternatively include, glidants, e.g. silica.

The composition may be in form of a powder, granule or pellets or in unit dosage form, for example as a tablet or capsule. The compositions of the present invention are well-adapted for encapsulation into an orally administrable capsule shell, particularly a hard gelatin shell. Alternatively the compositions may be compacted into tablets.

The tablets may optionally be coated, for instance with talc or a polysaccharide (e.g. cellulose) or hydroxypropylmethylcellulose coating.

The composition may also additionally comprise disintegrants. Examples of disintegrants are, for example, crosscarmellose cellulose, crosspovidone and sodium starch glycolate.

The core tablet compositions may also include, or alternatively include, surfactants, e.g. sodium lauryl sulphate, docusate sodium.

The core tablet compositions may also include, or alternatively include, gas producers, e.g. sodium bicarbonate, citric acid.

The composition may comprise a release rate controlling additive. For example, the drug may be held within a hydrophobic polymer matrix so that it is gradually leached out of the matrix upon contact with body fluids.

Alternatively, the drug may be held within a hydrophilic matrix which gradually or rapidly dissolves in the presence of body fluid. The tablet core may comprise two or more layers having different release properties. The layers may be hydrophilic, hydrophobic or a mixture of hydrophilic and hydrophobic layers. Adjacent layers in a multilayer tablet core may be separated by an insoluble barrier layer or hydrophilic separation layer. An insoluble barrier layer may be formed of materials used to form the insoluble casing. A hydrophilic separation layer may be formed from a material more soluble than the other layers of the tablet core so that as the separation layer dissolves the release layers of the tablet core are exposed.

Suitable release rate controlling polymers include polymethacrylates, ethylcellulose, hydroxypropylmethylcellulose, methylcellulose, hydroxyethylcellulose, hydroxypropylcellulose, sodium carboxymethylcellulose, calcium carboxymethylcellulose, acrylic acid polymer, polyethylene glycol, polyethylene oxide, carrageenan, cellulose acetate, zein etc.

The composition may additionally include materials which swell on contact with aqueous liquids, and which may be included in the composition, include polymer materials include from cross-linked sodium carboxymethylcellulose, cross-linked hydroxypropylcellulose, high molecular weighthydroxypropylcellulose, carboxymethylamide, potassium methacrylatedivinylbenzene copolymer, polymethylmethacrylate, cross-linked polyvinylpyrrolidone and high molecular weight polyvinylalcohols.

In one embodiment of the present invention, the composition includes a silicon dioxide.

The microcrystalline cellulose may act as a diluent, carrier, filler or bulking agent, and may suitably be Avicel®. The size of the particles of the microcrystalline cellulose may vary.

The use of microcrystalline cellulose composition may assist in promoting uniform distribution of the S1P receptor agonist throughout the microcrystalline cellulose in the composition. A higher surface area may be achieved by providing a microcrystalline cellulose preparation consisting of particles having a smaller mean size and/or a rougher surface on each particle.

The use of micronized microcrystalline cellulose, e.g. with a mean particle size of 30 µm or less, has also been found to improve compressibility and hardness of tablets formed from the composition.

The composition preferably contains 75 to 99.99% by weight of the microcrystalline cellulose, e.g. 85 to 99.9%, e.g 90 to 99.5% by weight, based on the total weight of the composition.

Typically sugar alcohols include lactose, sucrose, dextrose, mannitol or sorbitol.

Compositions of the present invention may be in the form of, for example, tablets, capsules, caplets, lozenges, pills, mini-tablets, pellets, beads and granules.

Where the solid composition is in the form of pellets or granules, these may, after application of the coating as described hereinafter, be used as such or to fill capsules, e.g. hard gelatine capsules or other storage means, for example sachets prior to administration.

Pellets and granules may be from 2mm to 0.3mm in diameter, for example, a "normal pellet" has a size of 1-0.6mm and a "bead pellet" has a size of 0.4 to 0.8mm

In one embodiment of the present invention, the oral composition described herein promotes the absorption and distribution of the S1 P agonist and/or modulator through the blood brain barrier and into the brain.

### S1P receptor agonists and/or modulators

S1P receptor agonists are typically sphingosine analogues, such as 2-substituted 2-aminopropane-1,3-diol or 2-amino-propanol derivatives. Examples of appropriate S1P receptor agonists are, for example:

Compounds as disclosed in EP627406A1, e.g. a compound of formula I 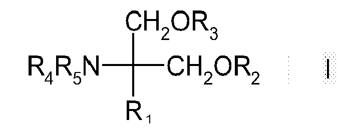 wherein
- R₁: is a straight- or branched (C₁₂₋₂₂) carbon chain,
which may have in the chain a bond or a hetero atom selected from a double bond, a triple bond, O, S, NR₆, wherein R₆ is H, alkyl, aralkyl, acyl or alkoxycarbonyl, and carbonyl; and/or
which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
- R₁: is a phenylalkyl wherein alkyl is a straight- or branched (C₆₋₂₀)carbon chain; or a phenylalkyl wherein alkyl is a straight- or branched (C₁₋₃₀)carbon chain wherein said phenylalkyl is substituted by
a straight- or branched (C₆₋₂₀)carbon chain optionally substituted by halogen,
a straight- or branched (C₆₋₂₀)alkoxy chain optionally substitued by halogen,
a straight- or branched (C₆₋₂₀)alkenyloxy;
phenylalkoxy, halophenylalkoxy, phenylalkoxyalkyl, phenoxyalkoxy or phenoxyalkyl;
cycloalkylalkyl substituted by a straight- or branched (C₆₋₂₀)alkyl chain;
heteroarylalkyl substituted by a straight- or branched (C₆₋₂₀)alkyl chain;
heterocyclic alkyl wherein said alkyl is a straight- or branched (C₆₋₂₀)carbon chain; or
heterocyclic alkyl substituted by a straight- or branched (C₂₋₂₀)alkyl chain,
and wherein the alkyl moiety may have in the carbon chain, a bond or a heteroatom selected from a double bond, a triple bond, O, S, sulfinyl, sulfonyl, or NR₆, wherein R₆ is as defined above;
and as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and
each of R₂, R₃, R₄ and R₅, independently, is H, C₁₋₄ alkyl or acyl
or a pharmaceutically acceptable salt thereof;

Compounds as disclosed in EP 1002792A, e.g. a compound of formula II 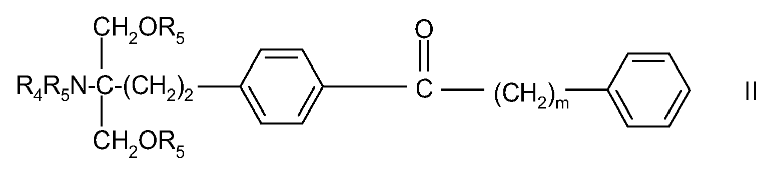 wherein
m is 1 to 9; and
each of R₂, R₃, R₄ and R₅, independently, is H, alkyl or acyl;
or a pharmaceutically acceptable salt thereof;

Compounds as disclosed in EP0778263 A1, e.g. a compound of formula III 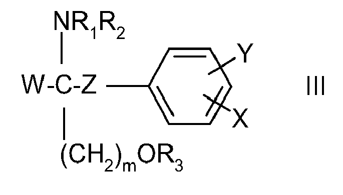 wherein
- W: is H; straight chain or branched (C₁₋₆)alkyl, (C₂₋₆)alkenyl or (C₂₋₆)alkynyl; unsubstituted or by OH substituted phenyl; R₄O(CH₂)ₙ; or straight chain or branched (C₁₋₆)alkyl substituted by 1 to 3 substituents selected from the group consisting of halogen, cycloalkyl, phenyl or phenyl substituted by OH;
- X: is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of alkyl, OH, alkoxy, acyloxy, amino, alkylamino, acylamino, oxo, haloalkyl, halogen, unsubstituted phenyl or phenyl substituted by 1 to 3 substituents selected from the group consisting of alkyl, OH, alkoxy, acyl, acyloxy, amino, alkylamino, acylamino, haloalkyl and halogen;
- Y: is H, alkyl, OH, alkoxy, acyl, acyloxy, amino, alkylamino, acylamino, haloalkyl or halogen, Z is a single bond or a straight chain alkylene having a number or carbon atoms of q,
each of p and q, independently, is an integer of 1 to 20, with the proviso of 6≤p+q≤23,
m is 1, 2 or 3,
n is 2 or 3,
each of R₁, R₂, R₃ and R₄, independently, is H, alkyl or acyl,
or a pharmaceutically acceptable salt thereof.

Compounds as disclosed in WO02/18395, e.g. a compound of formula IVa or IVb 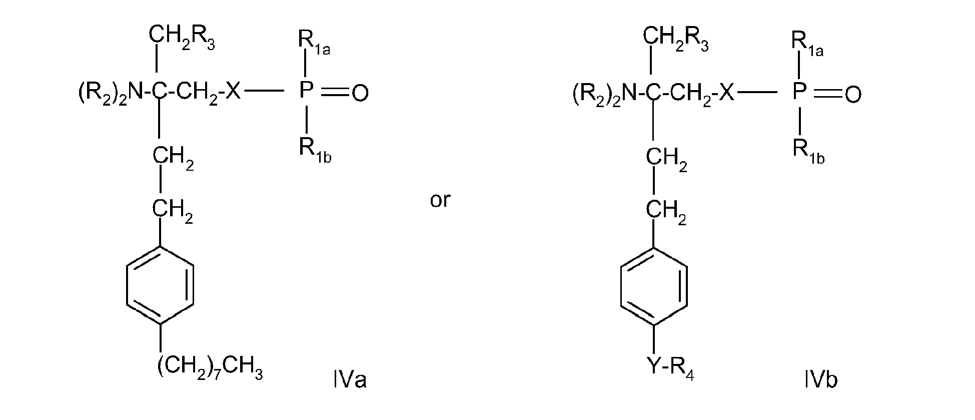 wherein
- X: is O, S, NR₁ or a group -(CH₂)ₙ-, which group is unsubstituted or substituted by 1 to 4 halogen;
- n: is 1 or 2,
- R₁: is H or (C₁₋₄)alkyl, which alkyl is unsubstituted or substituted by halogen;
- R₁ₐ: is H, OH, (C₁₋₄)alkyl or O(C₁₋₄)alkyl wherein alkyl is unsubstituted or substituted by 1 to 3 halogen;
- R_{1b}: is H, OH or (C₁₋₄)alkyl, wherein alkyl is unsubstituted or substituted by halogen;
- each R₂: is independently selected from H or (C₁₋₄)alkyl, which alkyl is unsubstituted or substitued by halogen;
- R₃: in case of a compound of formula lVa is H, OH, halogen or O(C₁₋₄)alkyl wherein alkyl is unsubstituted or substituted by halogen;
- R₃: in case of a compound of formula IVb is H, OH, halogen, (C₁₋₄)alkyl wherein alkyl is unsubstituted or substituted by hydroxy, or O(C₁₋₄)alkyl wherein alkyl is unsubstituted or substituted by halogen,
- Y: is -CH₂-, -C(O)-, -CH(OH)-, -C(=NOH)-, O or S,
- R₄: is (C₄₋₁₄)alkyl or (C₄₋₁₄)alkenyl;
or a pharmaceutically acceptable salt or hydrate thereof;

Compounds as disclosed in WO 02/06268 or JP-14316985, e.g. a compound of formula VII 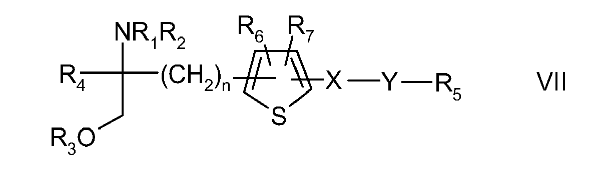 wherein
each of R₁ and R₂, independently, is H or an amino-protecting group;
R₃ is hydrogen or a hydroxy-protecting group;
R₄ is (C₁₋₆)alkyl;
n is an integer of 1-6;
- X: is ethylene, vinylene, ethynylene, a group having a formula - D-CH₂- (wherein, D is carbonyl, a group having a formula -CH(OH)-, O, S or N; aryl or aryl substituted by three members selected from group a as defined hereinafter;
- Y: is single bond, C₁₋₁₀alkylene, C₁₋₁₀alkylene which is substituted by one to three substituents selected from groups a and b, C₁₋₁₀alkylene having O or S in the middle or end of the carbon chain, or C₁₋₁₀alkylene having O or S in the middle or end of the carbon chain which is substituted by one to three substituents selected from groups a and b;
- R₅: is hydrogen, cycloalkyl, aryl, heterocycle, cycloalkyl substituted by one to three members selected from groups a and b, aryl substituted by one to three members selected from groups a and b, or heterocycle substituted by one to three members selected from groups a and b; and
each of R₆ and R₇, independently, is H or a substituent selected from group a;
<group a> is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-lower alkylamino, lower aliphatic acylamino, cyano and nitro;
<group b> is cycloalkyl, aryl, heterocycle, each being optionally substituted by up to three substituents selected from group a;
with the proviso that when R₅ is hydrogen, Y is either a single bond or linear C₁₋₁₀ alkylene,
e.g. (2R)-2-amino-4-[3-(4-cyclohexyloxybutyl)benzo[b]thien-6-yl]-2-methylbutan-1-ol,
or a pharmacologically acceptable salt or ester thereof.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy. Acyl may be a residue R-CO-, wherein R is C₁₋₆alkyl, C₃₋₆cycloalkyl, phenyl or phenyl-C₁₋₄alkyl.

Preferred compounds of formula I are those wherein R₁ is a straight or branched, preferably straight, chain alkyl having 13-20 carbon atoms, optionally substituted by nitro, halogen, amino, hydroxy or carboxy, and, more preferably those wherein R₁ is phenylalkyl substituted by a straight or branched C₆₋₁₄alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

A preferred compound of formula I is 2-amino-2-tetradecyl-1,3-propanediol. A particularly preferred S1 P receptor agonist of formula I is 2-amino-2-[2-(4-octylphenylethyl)]propane-1,3-diol in free form or in a pharmaceutically acceptable salt form (referred to hereinafter as Compound A), e.g. the hydrochloride, i.e. FTY720, as shown:

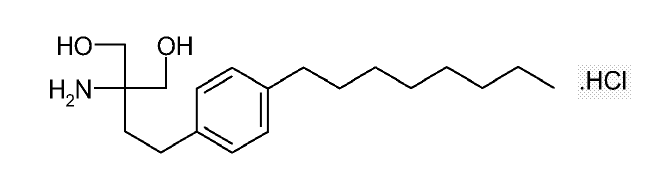

A preferred compound of formula II is one wherein each of R₂ to R₅ is H and m is 4, i.e. 2-amino-2-{2-[4-(1-oxo-5-phenylpentyl)phenyl]ethyl}propane-1,3-diol (referred to hereinafter as Compound B), in free form or in a pharmaceutically acceptable salt form, e.g. the hydrochloride.

A preferred compound of formula lVa is the Compound A-phosphate (R₂ is H, R₃ is OH, X is O, R₁ₐ and R_{1b} are OH). A preferred compound of formula V is Compound B-phosphate (R₁ is CH₂OH, R₃ is H, X is O, m is 1, R₂ is phosphate and R is 2-[4-(1-oxo-5-phenylpentyl)phenyl]ethyl).

When the compounds of formulae I to VII have one or more asymmetric centers in the molecule, the various optical isomers, as well as racemates, diastereoisomers and mixtures thereof are embraced.

Examples of pharmaceutically acceptable salts of the compounds of formulae I to VII include salts with inorganic acids, such as hydrochloride, hydrobromide and sulfate, salts with organic acids, such as acetate, fumarate, maleate, benzoate, citrate, maleate, methanesulfonate and benzenesulfonate salts, or, when appropriate, salts with metals, such as sodium, potassium, calcium and aluminium, salts with amines, such as triethylamine and salts with dibasic amino acids, such as lysine. The compounds and salts of the present invention encompass hydrate and solvate forms.

The composition of the present invention may comprise one or more salts and/or free acid of the S1 P agonists and//or modulator.

The composition of the invention preferably contains 0.01 to 20% by weight of S1P receptor agonists, more preferably 0.1 to 10%, e.g. 0.5 to 5% by weight, based on the total weight of the composition.

Where the pharmaceutical capsule is in unit dosage form, each unit dosage will suitably contain 0.5 to 10 mg of the S1 P receptor agonist.

The compositions of the invention may show good stability characteristics as indicated by standard stability trials, for example having a shelf life stability of up to one, two or three years, and even longer. Stability characteristics may be determined, e.g. by measuring decomposition products by HPLC analysis after storage for particular times, at particular temperatures, e.g. 20°, 40° or 60°C.

The pharmaceutical compositions of the present invention may be produced by standard processes, for instance by conventional mixing, granulating, sugar-coating, dissolving or lyophilizing processes. Procedures which may be used are known in the art, e.g. those described in L. Lachman et al. The Theory and Practice of Industrial Pharmacy, 3rd Ed, 1986, H. Sucker et al, Pharmazeutische Technologie, Thieme, 1991, Hagers Handbuch der pharmazeutischen Praxis, 4th Ed. (Springer Verlag, 1971) and Remington's Pharmaceutical Sciences, 13th Ed., (Mack Publ., Co., 1970) or later editions.

In one aspect, the present invention relates to a process for producing a pharmaceutical composition, comprising:
(a) mixing an S1 P receptor agonist with a microcrystalline cellulose, e.g. Avicel®,;
(b) milling and/or granulating the mixture obtained in (a); and
(c) optionally mixing the milled mixture obtained in (b) with a lubricant.

By using this process, a preparation having a good level of content and blend uniformity (i.e. a substantially uniform distribution of the S1 P receptor agonist throughout the composition), dissolution time and stability is obtained.

The S1P receptor agonist, e.g. 2-amino-2-[2-(4-octylphenyl)ethyl]propane-1,3-diol, hydrochloride, may optionally be micronized, and/or pre-screened, e.g. with a 400 to 500 µm mesh screen, before step (a) in order to remove lumps. The mixing step (a) may suitably comprise blending the S1 P receptor agonist and the microcrystalline cellulose, e.g. Avicel®, in any suitable blender or mixer for e.g. 100 to 400 revolutions.

The process may be carried out by dry mixing the components. In this embodiment the milling step (b) may suitably comprise passing the mixture obtained in (a) through a screen, which preferably has a mesh size of 400 to 500 µm. Process step (a) may comprise the step of mixing the total amount of S1P receptor agonist at first with a low amount of microcrystalline cellulose, e.g. Avicel®, , e.g. from 5 to 25% by weight of the total weight of microcrystalline cellulose, e.g. Avicel®, , in order to form a pre-mix. Subsequently the remaining amount of microcrystalline cellulose, e.g. Avicel®, is added to the pre-mix. Step (a) may also comprise the step of adding a binder solution, e.g. methylcellulose and/or xylitol, e.g. an aqueous solution, to the mixture.

The milled mixture obtained in (b) may optionally be blended once more before mixing with the lubricant. The lubricant, e.g. magnesium stearate, is preferably pre-screened, e.g. with a 800 to 900 µm screen, before mixing.

Alternatively, a wet granulation process is employed. In this embodiment, the S1 P receptor agonist is preferably first dry-mixed with the desired microcrystalline cellulose, e.g. Avicel®, and the obtained microcrystalline cellulose, e.g. Avicel®, /S1P receptor agonist mixture is then dry-mixed with a binder such as hydroxypropyl cellulose or hydroxypropylmethyl cellulose. Water is then added and the mixture granulated, e.g. using an automated granulator. The granulation is then dried and milled.

If desirable, an additional amount of binder may be added in step (c) to the mixture obtained in (b).

The process may comprise a further step of tabletting or encapsulating the mixture obtained in (c), e.g. into a hard gelatin capsule using an automated encapsulation device. The capsules may be coloured or marked so as to impart an individual appearance and to make them instantly recognizable. The use of dyes can serve to enhance the appearance as well as to identify the capsules. Dyes suitable for use in pharmacy typically include carotinoids, iron oxides, and chlorophyll. Preferably, the capsules of the invention are marked using a code.

The pharmaceutical compositions of the present invention are useful, either alone or in combination with other active agents, for the treatment and prevention of conditions e.g. as disclosed in US 5,604,229, WO 97/24112, WO 01/01978, US 6,004,565, US 6,274,629 and JP-14316985, the contents of which are incorporated herein by reference.

In particular, the pharmaceutical compositions are useful for:
a) treatment and prevention of organ or tissue transplant rejection, for example for the treatment of the recipients of heart, lung, combined heart-lung, liver, kidney, pancreatic, skin or corneal transplants, and the prevention of graft-versus-host disease, such as sometimes occurs following bone marrow transplantation; particularly in the treatment of acute or chronic allo- and xenograft rejection or in the transplantation of insulin producing cells, e.g. pancreatic islet cells;
b) treatment and prevention of autoimmune disease or of inflammatory conditions, e.g. multiple sclerosis, arthritis (for example rheumatoid arthritis), inflammatory bowel disease, hepatitis, etc.;
c) treatment and prevention of viral myocarditis and viral diseases caused by viral mycocarditis, including hepatitis and AIDS.

The invention is, in one embodiment, related to the treatment of inflammatory conditions. In one example, the invention is related to compositions capable of regulating and/or suppressing mast cell activation and secretion for the relief of inflammatory conditions, e.g., in the brain as in multiple sclerosis.

There is also provided a method of protecting multiple sclerosis subjects against neurodegerative brain inflammation, comprising the administration to said subjects the a composition as described herein, for example a composition comprising an S1P agonist and/or modulator.

The invention will now be described with reference to the following specific embodiments.

### Example 1

Micronized Compound 2-amino-2-[2-(4-octylphenyl)ethyl]propane-1,3-diol, hydrochloride salt (FTY720), is screened mixed with the microcrystalline cellulose agent, e.g. Avicel PH 102. The mixture is then milled in a Frewitt MGI device (Key International Inc. USA) using a 30 mesh screen. Magnesium stearate is screened using a 20 mesh screen and blended with the FTY720/cellulosemixture. Crosscarmellose is the blended to produce a product composition.

An example for a 6 mm round, 80 mg tablet core obtained by direct compression is shown below:

| Ingredient | mg/dose |
|---|---|
| FTY720 HCl | 1.40 |
| Microcrystalline cellulose, e.g. Avicel PH 102 | 73.80 |
| Magnesium stearate | 0.80 |
| Crosscarmellose | 4.00 |

### Example 2

Micronized Compound A, e.g. 2-amino-2-[2-(4-octylphenyl)ethyl]propane-1,3-diol, hydrochloride salt (FTY720), is screened and 116.7 g of the screened compound is mixed with 9683.3 g of a microcrystalline cellulose agent. The mixture is then milled in a Frewitt MGI device (Key International Inc. USA) using a 30 mesh screen. Magnesium stearate is screened using a 20 mesh screen and 200 g of the screened compound blended with the FTY720 mixture to produce a product composition.

The product composition is then compacted on a tablet press using a 7 mm die to form 120 mg tablets, each containing:

| | |
|---|---|
| Compound A, e.g. FTY720 * | 1.4 mg |
| Microcrystalline cellulose, e.g. Avicel PH 102 | 116.2 mg |
| Magnesium stearate | 2.4 mg |
| | |
| Total | 120 mg |

| | |
|---|---|
| * 1 mg of Compound A in free form is equivalent to 1.12 mg of FTY720. | |

### Example 3

In a further example, the process of example 2 is repeated except that the magnesium stearate is replaced by Cutina® (hydrogenated castor oil).

### Example 4

Compound A, e.g. FTY720, and Microcrystalline cellulose, e.g. Avicel PH 102 are each screened separately using an 18 mesh screen. 1.9 g screened FTY720 is mixed with 40 g screened microcrystalline cellulose agent for 120 revolutions in a blender at 32 rpm. The FTY720 mixture is then screened through a 35 mesh screen.

The screened FTY720 mixture is added to a granulator along with a further 340.1 g Microcrystalline cellulose, e.g. Avicel PH 102 and 12 g hydroxypropylcellulose. The mixture is mixed for 3 minutes. Water is then added at a rate of 100 ml/minute and the mixture granulated for 2 minutes. The granulation is transferred into a tray dryer and dried at 50°C for 150 minutes.

The mixture is then milled in a Frewitt MGI device using a 35 mesh screen. Magnesium stearate is screened and 6 g of the screened compound is blended for 90 revolutions at 32 rpm with the FTY720 mixture to produce a product composition showing a substantially uniform distribution of the S1 P receptor agonist throughout the Microcrystalline cellulose, e.g. Avicel PH 102 in the blend.

The product composition is then filled into size 3 hard gelatin shells on an H & K 400 encapsulation device. 120 mg of the product composition is added to each capsule. Therefore each capsule contains:

| | |
|---|---|
| FTY720 * | 0.56 mg |
| Microcrystalline cellulose | 114.04mg |
| Hydroxypropylcellulose | 3.6 mg |
| Magnesium stearate | 1.8 mg |
| | |
| Total | 120 mg |

### Example 5

In a further example, the process of example 4 is repeated except that the magnesium stearate is replaced by Cutina® (hydrogenated castor oil).

### Example 6

In a further example, the process of example 4 is repeated except that the hydroxypropyl cellulose is replaced by hydroxypropylmethyl cellulose.

### Example 7

Micronized Compound A, e.g. FTY720, is screened using a 425 µm (40 mesh) screen. 58.35 g of the screened compound is mixed with 4841.65 g Microcrystalline cellulose, e.g. Avicel PH 102 in a 25L Bohle bin blender for 240 blending revolutions. The mixture is then milled in a Frewitt MGI device using a 425 µm mesh screen, and the milled mixture is blended once more. Magnesium stearate is screened and 100 g of the screened compound is blended with the FTY720 mixture to produce a product composition showing a substantially uniform distribution of the S1 P receptor agonist throughout the blend.

The product composition is then filled into size 3 hard gelatin shells on an H & K 400 encapsulation device. 120 mg of the product composition is added to each capsule. Therefore each capsule contains:

| | |
|---|---|
| FTY720 * | 1.4 mg |
| Microcrystalline cellulose | 116.2 mg |
| Magnesium stearate | 2.4 mg |
| | |
| Total | 120 mg |

### Examples 8 and 9

In further examples, capsules are prepared as described in example 7, except that each capsule contains each component in the following amounts:

| | Example 8 | Example 9 |
|---|---|---|
| FTY720 * | 2.8 mg | 5.6 mg |
| Microcrystalline cellulose | 114.8 mg | 112 mg |
| Magnesium stearate | 2.4 mg | 2.4 mg |
| | | |
| Total | 120 mg | 120 mg |

### Examples 10 to 12

In further examples, capsules are prepared as described in examples 7 to 9, except that the magnesium stearate is replaced in each case by Cutina® (hydrogenated castor oil).

### Examples 13 to 23

In further examples, capsules or tablets are prepared as described in examples 1 to 11, except that FTY720 is replaced in each case by 2-amino-2-{2-[4-(1-oxo-5-phenylpentyl)phenyl]ethyl}propane-1,3-diol hydrochloride.

### Examples 24 to 26

Pharmaceutical compositions containing the following ingredients are produced:

| | Example 24 | Example 25 | Example 26 |
|---|---|---|---|
| FTY720 | 5 g | 10 g | 100 g |
| Microcrystalline cellulose | 991 g | 986 g | 897 g |
| Methylcellulose SM-25 | 4 g | 4 g | 3 g |
| | | | |
| Total | 1000 g | 1000 g | 1000 g |

The FTY720 and a proportion of the Microcrystalline cellulose, e.g. Avicel PH 102 equal to twice the weight of the FTY720 are mixed in a Microspeed Mixer MS-5 type (Palmer, USA) for 2 minutes at 1200 rpm. The remaining Microcrystalline cellulose is added to the mixture and mixed for another 2 minutes. 80 or 60 milliliters of 5% methylcellulose SM-25 solution is supplied from a hopper and granulated under the same conditions. The mixture is extruded through a screen with 0.4 mm apertures using an extruder RG-5 type. The extruded material is dried at 65°C by a fluidized-bed granulator STREA I Type (Patheon, Canada) and then sieved through a 24 mesh sieve. Fine particles which pass through a 60 mesh sieve are removed. The obtained fine granules are filled into capsules by a Zuma capsule-filling machine (100 mg per capsule).

## Claims

1. A solid pharmaceutical composition suitable for oral administration, comprising:
(a) a S1 P receptor agonist; and
(b) a microcrystalline cellulose
in the absence of a sugar alcohol.

2. A composition according to claim 1, wherein the S1P receptor agonist comprises 2-amino-2-[2-(4-octylphenyl)ethyl]propane-1,3-diol or 2-amino-2-{2-[4-(1-oxo-5-phenylpentyl)-phenyl]ethyl}propane-1,3-diol or a pharmaceutically acceptable salt thereof.

3. A composition according to claim 1 or claim 2, wherein the microcrystalline cellulose comprises Avicel®.

4. A composition according to claim 1, 2 or 3, further comprising a lubricant.

5. A composition according to claim 4, wherein the lubricant comprises magnesium stearate.

6. A composition according to any preceding claim, comprising 0.5 to 5% by weight of the S1P receptor agonist.

7. A composition according to any preceding claim, comprising 90 to 99.5% by weight of the microcrystalline cellulose.

8. A composition according to any preceding claim, comprising 1.5 to 2.5% by weight of the lubricant.

9. A composition according to any preceding claim, further comprising hydroxypropyl cellulose, hydroxypropylmethyl cellulose or methyl cellulose.

10. A composition according to any preceding claim, in the form of a granule.

11. A composition according to any preceding claim, in the form of a tablet.

12. A composition according to any preceding claim, in the form of a capsule.

13. A hard gelatin capsule comprising a S1P receptor agonist and a microcrystalline cellulose in the absence of a sugar alcohol.

14. A method of treating a subject in need of immunosuppression, comprising administering to the subject a composition according to any of claims 1 to 12.

15. Use of a pharmaceutical composition according to any of claims 1 to 12 for the preparation of a medicament for the prevention or treatment of organ or tissue transplant rejection, or for the prevention or treatment of an inflammatory or autoimmune disease.

16. A process for producing a pharmaceutical composition according to any of claims 1 to 12, comprising the steps:
(a) mixing S1 P receptor agonist with a microcrystalline cellulose, e.g. Avicel®, ;
(b) milling the mixture obtained in (a); and
(c) mixing the milled mixture obtained in (b) with a lubricant.

17. A method of protecting multiple sclerosis subjects against neurodegerative brain inflammation, comprising the administration to said subjects the composition of any of claims 1 to 12.
